# EUROPEAN PATENT SPECIFICATION

(11) **EP 3 003 442 B1**
(45) Date of publication and mention of the grant of the patent: **30.12.2020**
(21) Application number: 14805039.6
(22) Date of filing: 29.05.2014
(51) Int. Cl.: A61M 5/142, A61M 5/168, A61M 5/36, G01N 29/02, G01N 29/34, G01N 29/42, H03M 1/12

(54) **INFUSION SYSTEM AND METHOD OF USE WHICH PREVENTS OVER-SATURATION OF AN ANALOG-TO-DIGITAL CONVERTER**
INFUSIONSSYSTEM UND VERFAHREN ZUR VERWENDUNG ZUR VERHINDERUNG DER ÜBERSÄTTIGUNG EINES ANALOG-DIGITAL-WANDLERS
SYSTÈME DE PERFUSION ET PROCÉDÉ D'UTILISATION ÉVITANT LA SURSATURATION D'UN CONVERTISSEUR ANALOGIQUE-NUMÉRIQUE

(30) Priority: 29.05.2013 US 201361828408 P
(43) Date of publication of application: 13.04.2016
(73) Proprietor: ICU Medical, Inc., San Clemente, CA 92673 (US)
(72) Inventor: DUMAS, John H., Libertyville, Illinois 60048 (US); KOTNIK, Paul T., Commerce Township, Michigan 48382 (US); SUR, Kunal, Evanston, Illinois 60202 (US); BELKIN, Anatoly S., Glenview, Illinois 60026 (US); RUCHTI, Timothy L., Gurnee, Illinois 60031 (US)
(74) Representative: Invent Horizon IP
(86) International application number: PCT/US2014/039986
(87) International publication number: WO 2014/194065

(56) References cited:
- WO-A1-00/41621
- WO-A1-96/41156
- WO-A1-98/44320
- WO-A1-2011/017778
- US-A- 4 607 520
- US-A- 5 455 423
- US-A- 5 469 851
- US-A- 5 533 389
- US-A1- 2005 192 529
- US-A1- 2010 212 407
- US-A1- 2010 212 407
- US-B1- 6 212 936
- US-B1- 7 678 048

## Description

### FIELD OF THE DISCLOSURE

This disclosure relates to an infusion system and method of use which prevents over-saturation of an analog-to-digital converter being used to determine whether air is present in the infusion system.

### BACKGROUND

The Symbiq™ infusion system, made by Hospira, Inc., previously detected whether air was present in the infusion system by operating one or more sensors at their resonant frequency, which was determined and set during calibration. The one or more sensors were used to transmit and receive a signal through a fluid delivery line of the infusion system in order to determine, based on the strength of the signal that propagated through the fluid delivery line, whether air, fluid, or some combination thereof was disposed in the fluid delivery line. Signals propagate better through liquid fluid than through air. The resonant frequency of the one or more sensors is the frequency at which the output of the signal transmitted from the one or more sensors is maximized for a given transfer medium such as for the infusion fluid contained within the fluid delivery line of the infusion system. Thus, conventional wisdom suggested that the one or more sensors would be most effective at their resonant frequency. However, analog-to-digital converters are used to convert the received analog signal from analog to digital in order for a processor to determine, based on the converted digital signal, whether air, fluid, or some combination thereof is disposed in the fluid delivery line of the infusion system. It has been observed or discovered by the Applicants that under certain conditions, analog-to-digital converters can become over-saturated if the output of the signal transmitted from the one or more sensors is too high.

A system and method is needed to overcome one or more issues of one or more of the existing systems and methods for detecting air in an infusion system.

US 2010/0212407 A1 discloses an air bubble detector that may be connected in line with an intravenous infusion line. In one embodiment of this document, the detector includes a microcontroller and a sensor circuit. The microcontroller determines the optimum range of scanning frequencies for the detector and operates the detector using this range of frequencies. When operating the detector, the controller determines optimal frequencies for scanning and performs scans, recording the duration or width of a signal received which exceeds a predetermined threshold. The controller may periodically scan the entire frequency to update the optimum frequency range used for scanning. The microcontroller sends a signal to the sensor circuit indicating a broad set of test frequencies desired for calibration. The sensor circuit sends back a return signal to the microcontroller based on the transmission of a test frequency range by a sensor through a fluid path. An optimum set of operational frequencies is chosen from the test frequencies. Once an optimum range of frequencies is chosen, the microcontroller sends the optimum set of frequencies to the sensor circuit and monitors the return signal from the circuit. For each return signal above an internal threshold value, one is added to a width counter. Based on the width count during the optimum set of frequencies, the microcontroller determines if bubbles are present and characterizes any bubbles in the fluid path. Once the optimum set of frequencies have been sent, the width counter is reset and the optimum set of frequencies are resent or the calibration repeated. A process of calibrating an air bubble sensor involves a calibration signal including a sweep of potential ultrasonic frequencies which is used by the sensor to detect air bubbles. The output of the air bubble detector is digital, analog, data or any combination of these. The microcontroller may use a PWM driver or digital to analog converter to generate an analog voltage or current output signal. The analog output could convey to the host system the various bubble sizes that are detectable by the sensor.

US 5533389 A discloses systems that control flow through a line, and in particular intravenous fluid delivery systems. This document discloses a system for controlling the flow of fluid through a line. Such a system includes first and second valves in the line between is located a chamber, part of which may be filled with fluid having a variable volume, and another part is filled with a measurement gas (such as air). The chamber may be isolated from the pressure effects in the rest of the line by closing both valves. The valves may also permit fluid to flow into or out of the chamber. The second portion of the chamber has a common boundary with the first part in such a way that the combined volume of the first and second parts is constant. There are also means for creating sound waves in the gas in the second part of the chamber, in order to measure the volume of the fluid in the first part, for directing the operation of the valves and for creating subsonic pressure variations to produce the desired flow. The volume measurement is achieved by acoustic volume measurement techniques. The system determines a frequency of self-resonance of the system, and, based on the frequency of self-resonance, the volume of liquid in the chamber. To determine the volume of fluid in the chamber the controller generates an electrical signal having a spectrum of frequencies, which is converted through a loudspeaker into sound waves in the chamber. The acoustic response to the signal input into the chamber is picked up by a microphone. Based on the response the controller determines whether a frequency in the spectrum causes the resonatable mass to resonate, and calculates, based on a determined resonant frequency, the volume of liquid in the chamber. To determine the resonant frequency the controller measures, at a frequency in the spectrum, the gain of the acoustic response relative to the electrical signal. The controller may also measure the phase angle of the acoustic response relative to the electrical signal, in order to determine the resonant frequency. The controller may also detect the presence of a gas-bubble in the liquid by examining the phase and gain information.

### SUMMARY

According to the invention, there is provided a method according to claim 1. The dependent claims define preferred embodiments of the invention.

Herein described is an infusion system for being operatively connected to a fluid delivery line and to an infusion container containing an infusion fluid. The infusion system includes a pump, at least one sensor, an analog-to-digital converter, at least one processor, and a memory. The at least one sensor is disposed adjacent to the fluid delivery line and configured to transmit and receive a signal to detect whether there is air in the fluid delivery line. The analog-to-digital converter is electronically connected to the at least one sensor for converting the received signal from analog to digital. The at least one processor is in electronic communication with the pump, the at least one sensor, and the analog-to-digital converter. The memory is in electronic communication with the at least one processor. The memory includes programming code for execution by the at least one processor. The programming code is configured to operate the at least one sensor at a modified frequency which is different than a resonant frequency of the at least one sensor in order to reduce an amplitude of an output of the signal transmitted from the at least one sensor to a level which is lower than a saturation level of the analog-to-digital converter.

Herein described is a method for detecting air in a fluid delivery line of an infusion system. In one step, infusion fluid is pumped through a fluid delivery line adjacent to at least one sensor. In another step, a signal is transmitted and received using the at least one sensor into and from the fluid delivery line. The at least one sensor is operated, using at least one processor, at a modified frequency which is different than a resonant frequency of the at least one sensor in order to reduce an amplitude of an output of the signal transmitted from the at least one sensor to a level which is lower than a saturation level of an analog-to-digital converter to avoid over-saturating the analog-to-digital converter. In an additional step, the signal received by the at least one sensor is converted from analog to digital using the analog-to-digital converter.

Herein described is a method for arranging and using an infusion system. In one step, a resonant frequency of at least one sensor is determined. In another step, a saturation level of an analog-to-digital converter is determined. In still another step, the at least one sensor is disposed adjacent to a fluid delivery line. In yet another step, a pump is connected to the fluid delivery line. In another step, the analog-to-digital converter is electronically connected to the at least one sensor. In an additional step, at least one processor is electronically connected to the pump, to the at least one sensor, and to the analog-to-digital converter. In still another step, the at least one processor is programmed to operate the at least one sensor at a modified frequency which is different than the resonant frequency of the at least one sensor in order to reduce an amplitude of an output of a signal transmitted from the at least one sensor to a level which is lower than the saturation level of the analog-to-digital converter to avoid over-saturating the analog-to-digital converter.

The scope of the present disclosure is defined solely by the appended claims and is not affected by the statements within this summary.

### BRIEF DESCRIPTION OF THE DRAWINGS

The disclosure can be better understood with reference to the following drawings and description. The components in the figures are not necessarily to scale, emphasis instead being placed upon illustrating the principles of the disclosure.
Figure 1 illustrates a block diagram of an infusion system;
Figure 2 is a graph illustrating how shifting the frequency of a sensor of the infusion system of Figure 1 to a modified frequency which is different than the resonant frequency of the sensor may avoid over-saturating an electronic detection device of Figure 1;
Figure 3 illustrates a cross-section through a segment of fluid delivery line coupled to an electronic transmitting device, a transmitter portion of a sensor, a receiver portion of the sensor, and an electronic detection device;
Figure 4 illustrates a top view through the piezoelectric crystals of the transmitter portion of the sensor of Figure 3;
Figure 5 illustrates a flowchart of a method for arranging and using an infusion system;
Figure 6 illustrates a flowchart of a method for detecting air in a fluid delivery line of an infusion system;
Figure 7 is a graph illustrating five different curves showing for five different illustrative sensors, which could each be tried as the sensor in the infusion system of Figure 1, how their respective signal strength varies as their modified frequency varies; and
Figure 8 illustrates a flowchart of a method for determining the modified frequency of an infusion system.

### DETAILED DESCRIPTION

The description is not to be taken in a limiting sense, but is made merely for the purpose of illustrating the general principles of the disclosure, since the scope of the disclosure is defined by the appended claims. It is noted that the Figures are purely for illustrative purposes and are not to scale.

Applicants have discovered through testing of the Symbiq™ infusion system that when some sensors are operated at their resonant frequency the signal transmitted from the one or more sensors sometimes is over-saturated and causes the analog-to-digital converter to become over-saturated. Variation in sensor manufacturing and assembly, especially bonding of the piezo-electric element to the supporting body in an ultrasonic sensor, can be significant. Some piezo-electric elements may be extremely well bonded within the sensor assembly while others may have many gaps, voids or air bubbles in the bonding of the element to the sensor body. Sensors with few bonding imperfections may have high output amplitude, particularly at resonant frequency. Applicants have further discovered that this high amplitude, over-saturated signal sometimes reduces the accuracy and reliability of the analog-to-digital converters which may not correctly convert the received over-saturated signal. Applicants have additionally discovered that this could lead to errors in the infusion system's determination as to whether or not air is contained in the infusion system and the generation of alarms.

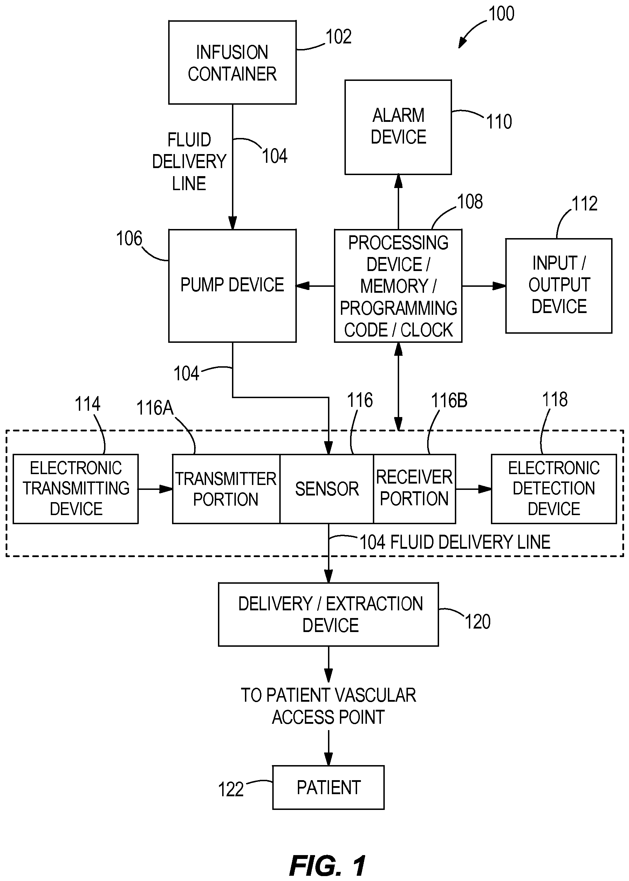

Figure 1 illustrates a block diagram of an infusion system 100. The infusion system 100 comprises: an infusion container 102; a fluid delivery line 104; a pump device 106; a processing device 108; an alarm device 110 that generates an audio, visual, other sensory signal or the like to a user; an input/output device 112; an electronic transmitting device 114; a sensor 116; an electronic detection device 118; and a delivery/extraction device 120. The infusion system 100 may comprise the Plum A+™, Gemstar™, Symbiq™, or other type of infusion system. The infusion container 102 comprises a container for delivering fluid such as IV fluid or a drug to the patient 122. The fluid delivery line 104 comprises one or more tubes, connected between the infusion container 102, the pump device 106, the sensor 116, and the delivery/extraction device 120, for transporting fluid from the infusion container 102, through the pump device 106, through the sensor 116, through the delivery/extraction device 120 to the patient 122. The fluid delivery line 104 may also be used to transport blood, extracted from the patient 122 using the delivery/extraction device 120, through the sensor 116 as a result of a pumping action of the pump device 106. The pump device 106 comprises a pump for pumping fluid from the supply container 102 or for pumping blood from the patient 122. The pump device 106 may comprise a plunger based pump, a peristaltic pump, or another type of pump.

The processing device 108 comprises at least one processor for processing information received from the electronic detection device 118 and/or the sensor 116 and for executing one or more algorithms to determine if air, fluid, or some combination thereof is located in the fluid delivery line 104 at the location of the sensor 116. The processing device 108 is in electronic communication with the pump device 106, the electronic transmitting device 114, the sensor 116, the electronic detection device 118, the input/output device 112, and the alarm device 110. The processing device 108 includes or is in electronic communication with a computer readable memory, containing programming code containing the one or more algorithms for execution by the at least one processor, and a clock.

The alarm device 110 comprises an alarm, triggered by the processing device 108, for notifying the clinician (also referred to as 'user' herein) as to the presence of air being disposed in the fluid delivery line 104 at the location of the sensor 116. The alarm device 110 may be configured to stop the pump device 106 prior to an air embolism being delivered through the fluid delivery line 104 and the delivery/extraction device 120 to the patient 122.

The input/output device 112 comprises a device which allows a clinician to input or receive information. The input/output device 112 allows a clinician to input or receive information regarding the infusion. For instance, the clinician may use the input/output device 112 to input or select a medication infusion program to be applied by the processing device 108, to set settings for the processing device 108 to apply in using the programming code containing the algorithm(s), or to input other type of information. The input/output device 112 may further output information to the clinician. In other embodiments, any of the information inputted into the input/output device 112 may be pre-installed into the programming code or the processing device 108.

The delivery/extraction device 120 comprises a patient vascular access point device for delivering fluid from the infusion container 102 to the patient 122, or for extracting blood from the patient 122. The delivery/extraction device 120 may comprise a needle, a catheter, or another type of delivery/extraction device. In other examples, the infusion system 100 of Figure 1 may be altered to vary the components, to take away one or more components, or to add one or more components.

The electronic transmitting device 114 comprises electronic circuitry, connected to the sensor 116, which transmits a signal from a transmitter portion 116A of the sensor 116, through the fluid delivery line 104, to a receiver portion 116B of the sensor 116. The transmitter portion 116A and the receiver portion 116B are disposed on opposed sides of the fluid delivery line 104. The receiver portion 116B of the sensor is electronically connected to the electronic detection device 118. The sensor 116 may comprise an air-in-line sensor for sensing, with the assistance of the electronic detection device 118 and the processing device 108, whether air, fluid, or some combination thereof is contained in the fluid delivery line 104. The sensor 116 is disposed adjacent to and/or connected to the fluid delivery line 104 distal of the pump device 106. In other comparative examples, the sensor 116 may be located proximal to the pump device 106 or may be located in both proximal and distal positions.

The transmitter and receiver portions 116A and 116B of the sensor 116 sense the presence of air, fluid, or some combination thereof within the fluid delivery line 104. The transmitter and receiver portions 116A and 116B of the sensor 116 comprise a transducer such as an ultrasonic sensor (in methods according to the invention), an acoustic sensor, an optical sensor, or another type of sensor (in comparative examples). Alternate arrangements of the sensor transmitter and receiver are possible and include both side-by-side arrangements and the use of a single transducer to both transmit and receive a reflected signal. In other examples, any number, configuration, and type of sensor(s) may be used.

The electronic detection device 118 comprises electronic circuitry, connected to the receiver portion 116B of the sensor 116, for receiving the signal transmitted from the electronic transmitting device 114, through the transmitter portion 116A of the sensor 116, through the fluid delivery line 104, to the receiver portion 116B of the sensor 116, to the electronic detection device 118. The electronic detection device 118 comprises an analog-to-digital converter which is electronically connected to the sensor 116 for converting the signal received by the receiver portion 116B of the sensor from analog to digital and communicating the digital reading to the processing device 108. The processing device 108 then determines, based on the digital reading, whether air, fluid, or some combination thereof is disposed in the fluid delivery line 104 at the sensor 116 by executing the programming code containing the one or more algorithms.

The programming code implemented by the processing device 108 is configured to operate the sensor 116 at a modified frequency which is different than a resonant frequency of the sensor 116 in order to reduce the amplitude of an output of the signal transmitted from the transmitter portion 116A of the sensor 116 to a level which is lower than a saturation level of the electronic detection device 118 comprising the analog-to-digital converter. The resonant frequency of the sensor 116 is the frequency at which the output of the signal transmitted from the transmitter portion 116A of the sensor 116 is maximized for a given transfer medium (such as the fluid delivery line 104 filled with infusion fluid).

By reducing the amplitude of the output of the signal transmitted from the transmitter portion 116A of the sensor 116 to a level which is lower than the saturation level of the electronic detection device 118 comprising the analog-to-digital converter, the reliability and accuracy of the processing device 108 detecting air, fluid, or some combination thereof in the fluid delivery line 104 is increased. This is because when the amplitude of an output of a signal transmitted from a sensor to an analog-to-digital converter is greater than a saturation level of the analog-to-digital converter, the accuracy and reliability of the analog-to-digital converter is reduced which may lead to errors in detecting air, fluid, or some combination thereof in the fluid delivery line. The resonant frequency of the sensor 116, the saturation level of the electronic detection device 118, and the amplitude of the output of the signal transmitted from the transmitter portion 116A of the sensor 116, set to be lower than the saturation level of the electronic detection device 118, each may be determined and/or set during calibration of the sensor 116 and the electronic detection device 118, or advantageously may be set at other times such as during use in the field.

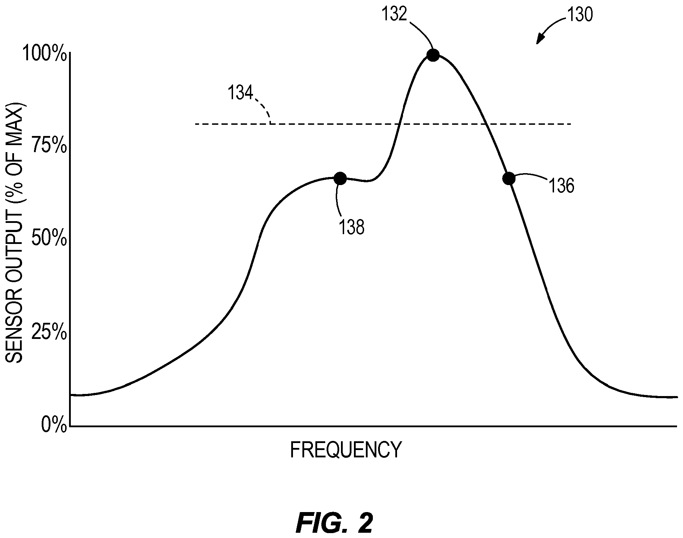

Figure 2 is a graph 130 illustrating how shifting the frequency of the sensor 116 of Figure 1 to a modified frequency which is different than the resonant frequency of the sensor 116 may avoid over-saturating the electronic detection device 118 of Figure 1. Frequency of the sensor 116 of Figure 1 is plotted on the X-axis of the graph 130. Output of the sensor 116 of Figure 1 expressed as a percentage of the maximum sensor output is plotted on the Y-axis of the graph 130. The resonant frequency 132 is the frequency at which the output of the sensor 116 of Figure 1 is maximized. The electronic detection device 118 of Figure 1 is over-saturated when the output of the sensor 116 of Figure 1 is above the saturation level 134. By reducing the output of the sensor 116 of Figure 1 to a percentage of maximum which is lower than the saturation level 134, over-saturation of the electronic detection device 118 of Figure 1 is avoided. As shown, this may be done by changing the frequency of the sensor 116 of Figure 1 to a modified frequency which is different than the resonant frequency 132 to reduce the output of the sensor 116 of Figure 1 to a level which is lower than the saturation level 134 of the electronic detection device 118 of Figure 1. For instance, at point 136 the frequency of the sensor 116 of Figure 1 has been increased beyond the resonant frequency 132 to reduce the output of the sensor 116 of Figure 1 to a level which is lower than the saturation level 134 of the electronic detection device 118 of Figure 1 to avoid over-saturating the electronic detection device 118. Similarly, at point 138 the frequency of the sensor 116 of Figure 1 has been decreased below the resonant frequency 132 to reduce the output of the sensor 116 of Figure 1 to a level which is lower than the saturation level 134 of the electronic detection device 118 of Figure 1 to avoid over-saturating the electronic detection device 118.

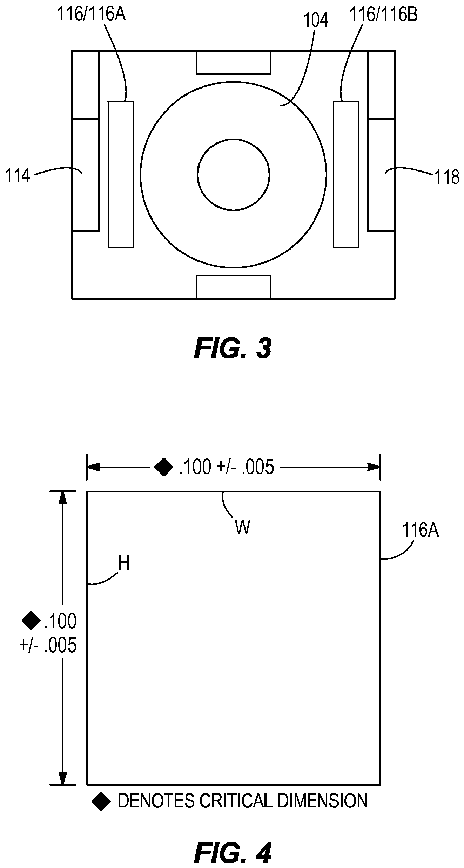

Figure 3 illustrates a cross-section through one embodiment of a segment of fluid delivery line 104 coupled to the electronic transmitting device 114, the transmitter portion 116A of the sensor 116, the receiver portion 116B of the sensor 116, and the electronic detection device 118. The transmitter and receiver portions 116A and 116B of the sensor 116 comprises piezoelectric crystals compressed against each side of the fluid delivery line 104 creating more surface area for uniform acoustic coupling and better signal to noise ratio. This arrangement of the transmitter and receiver portions 116A and 116B of the sensor 116 enables the transmission and detection of an ultrasonic signal through a target volume of the fluid delivery line 104. The electronic transmitting device 114 generates a nominal 5.25 MHz ultrasonic signal directed from the transmitter 116A portion of the sensor 116, through the fluid delivery line 104, to the receiver portion 116B of the sensor 116 connected to the electronic detection device 118. When fluid is present in the fluid delivery line 104 at the position of the sensor 116, the receiver portion 116B of the sensor 116 and the electronic detection device 118 generate a larger electrical signal than when air is present at the same position. Because of an inversion in the electronics of the electronic detection device 118, the software of the processing device 108 will receive a low signal when fluid is present at the location of the sensor 116, and a high signal when air is present at the location of the sensor 116. When a cassette is loaded into the pump device 106, the segment of the fluid delivery line 104 distal to the cassette is clamped into place in front of the sensor 116. This enables reliable and repeatable sensor performance over multiple cassettes.

Figure 4 illustrates a top view through the piezoelectric crystals of the transmitter portion 116A of the sensor 116 of Figure 3. As shown, the height H of the transmitter portion 116A comprises 0.100 inches and the width W of the transmitter portion 116A comprises 0.100 inches. The dimensions of the receiver portion 116B of the sensor 116 of Figure 1 are identical to the transmitter portion 116A. In other embodiments, the dimensions of the transmitter and receiver portions 116A and 116B of the sensor 116 may vary.

The ability of the ultrasonic signal to propagate from the transmitter portion 116A to the receiver portion 116B of the sensor 116 is governed by the acoustic impedance of the materials. The matching layers of the transducers of the transmitter and receiver portions 116A and 116B are designed to control the amplitude of the reflections at the piezo-matching layer and matching layer-fluid delivery line interfaces. The other significant component of the signal path is the fluid or air inside the fluid delivery line 104. The acoustic impedances (Za) @ 20°C of interest are as follows: water = 1.5 x 10⁶ kg / (m² s); PVC = 3.3 x 10⁶ kg / (m² s); and air = 413.2 kg / (m² s). Reflections of the ultrasonic signal occur at material boundaries and are governed by the differences in acoustic impedance. The reflection coefficient (RC) is defined as: RC = (Za - Za1) / (Za + Za1). A high RC indicates that the signal will not pass through the boundary. For the PVC to water interface, the RC = 0.375 which indicates that a majority of the signal will pass through the interface. For the PVC to air interface, the RC = 0.999 which indicates that a negligible, but non-zero portion of the signal energy will pass through the interface.

The electronic detection device 118 converts the signal received by the receiver portion 116B of the sensor 116 from an analog signal to a digital electrical signal as governed by the equation: Vout = λ Tpiezo σ / Drvr, where Vout = the electrical signal received by the receiver portion 116B of the sensor; λ = the strain on the piezo crystal due to the ultrasonic wave; σ = the stress on the piezo crystal due to the ultrasonic wave; Tpiezo = the thickness of the piezo crystal; Drvr = the mechanical displacement of the piezo by the ultrasonic crystal. Thus, when fluid is in the fluid delivery line 104, the receiver portion 116B of the sensor 116 is able to collect a large amount of ultrasonic energy since fluid is a better conductor than air. This appears as a low voltage at the analog-to-digital converter of the electronic detection device 118 because the signal received by the receiver portion 116B of the sensor 116 is inverted electrically. The position of the fluid (for instance a fluid droplet) inside the fluid delivery line 104 relative to the transmitter and receiver portions 116A and 116B of the sensor 116 also influences the amount of energy the receiver portion 116B of the sensor detects. When air is in the fluid delivery line 104, the receiver portion 116B of the sensor 116 collects little energy.

The processing device 108 of Figure 1 includes software components that receive the signal received by the receiver portion 116B of the sensor 116 and converted to a digital signal though the electronic detection device 118. The processing device 108 processes the received digital signal, and generates an alarm, using the alarm device 110 of Figure 1, when the one or more algorithms stored in the programming code indicates that an amount of air over the air threshold is present.

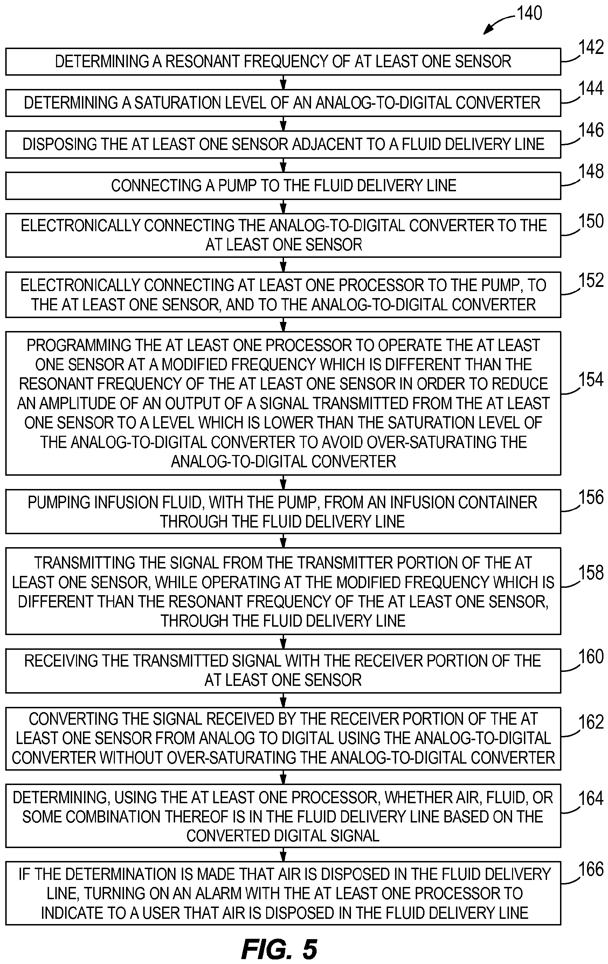

Figure 5 illustrates a flowchart of a method 140 for arranging and using an infusion system. The method 140 may utilize the infusion system of Figure 1. In other examples, the method 140 may utilize varying systems. In step 142, a resonant frequency of at least one sensor is determined. Step 142 may be done during calibration of the at least one sensor. In another example, step 142 may be done at a varying time such as when in use in the field. In step 144, a saturation level of an analog-to-digital converter is determined. Step 144 may be done during calibration of the analog-to-digital converter. Advantageously in another example, step 144 may be done at a varying time such as when in use in the field. In step 146, the at least one sensor is disposed adjacent to a fluid delivery line. In one example, step 146 may comprise disposing a transmitter portion of the at least one sensor and a receiver portion of the at least one sensor on opposed sides of the fluid delivery line. In step 148, a pump is connected to the fluid delivery line. In step 150, the analog-to-digital converter is electronically connected to the at least one sensor. In step 152, at least one processor is electronically connected to the pump, to the at least one sensor, and to the analog-to-digital converter.

In step 154, the at least one processor is programmed to operate the at least one sensor at a modified frequency which is different than the resonant frequency of the at least one sensor in order to reduce an amplitude of an output of a signal transmitted from the at least one sensor to a level which is lower than the saturation level of the analog-to-digital converter to avoid over-saturating the analog-to-digital converter. In one example, step 154 may be done during calibration of the at least one sensor. Advantageously in another embodiment, step 154 may be done at a varying time such as when in use in the field. In still another example, any or each of steps 142, 144, and 154 may be done prior to steps 146, 148, 150, and 152.

In step 156, infusion fluid is pumped, with the pump, from an infusion container through the fluid delivery line. In step 158, the signal is transmitted from the transmitter portion of the at least one sensor, while operating at the modified frequency which is different than the resonant frequency of the at least one sensor, through the fluid delivery line. In step 160, the transmitted signal is received with the receiver portion of the at least one sensor. In step 162, the signal received by the receiver portion of the at least one sensor is converted from analog to digital using the analog-to-digital converter without over-saturating the analog-to-digital converter. In step 164, a determination is made, using the at least one processor, whether air, fluid, or some combination thereof is in the fluid delivery line based on the converted digital signal. In step 166, if the determination is made in step 164 that air is disposed in the fluid delivery line, the at least one processor turns on an alarm to indicate to a user that air is disposed in the fluid delivery line. In one example, if the determination is made in step 164 that air is disposed in the fluid delivery line, then in step 166 the at least one processor turns on the alarm and shuts down the infusion system to stop the delivery of infusion fluid through the fluid delivery line. In other examples, the method 140 may be altered to vary the order or substance of any of the steps, to delete one or more steps, or to add one or more steps.

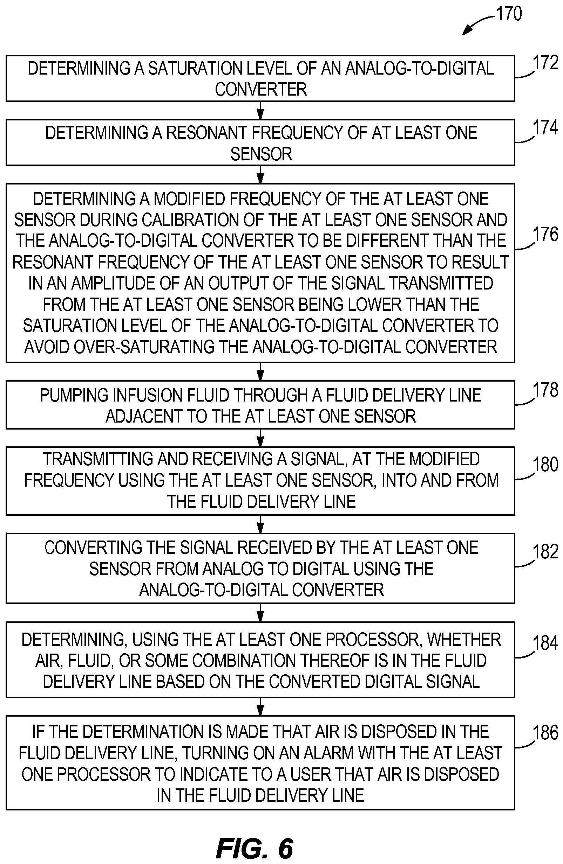

Figure 6 illustrates a flowchart of a method 170 for detecting air in a fluid delivery line of an infusion system. The method 170 may utilize the infusion system of Figure 1. In other examples, the method 170 may utilize varying systems. In step 172, a saturation level of an analog-to-digital converter may be determined. Step 172 may be done during calibration of the analog-to-digital converter. In other examples, step 172 may be done at a varying time such as when in use in the field. In step 174, a resonant frequency of at least one sensor is determined. Step 174 may be done during calibration of the at least one sensor. Advantageously in other examples, step 174 may be done at a varying time such as when in use in the field. In step 176, a modified frequency of the at least one sensor may be determined during calibration of the at least one sensor and the analog-to-digital converter to be different than the resonant frequency of the at least one sensor to result in an amplitude of an output of the signal transmitted from the at least one sensor being lower than the saturation level of the analog-to-digital converter to avoid over-saturating the analog-to-digital converter. In other examples, step 176 may be done at a varying time such as when in use in the field.

In step 178, infusion fluid is pumped through a fluid delivery line adjacent to the at least one sensor. In step 180, a signal is transmitted and received, using the at least one sensor, into and from the fluid delivery line. Step 180 further comprises the at least one sensor operating, using at least one processor, at the modified frequency which is different than the resonant frequency of the at least one sensor in order to reduce an amplitude of an output of the signal transmitted from the at least one sensor to a level which is lower than the saturation level of the analog-to-digital converter to avoid over-saturating the analog-to-digital converter. In one example, step 180 comprises transmitting the signal from a transmitter portion of the at least one sensor disposed on one side of the fluid delivery line, while operating at the modified frequency which is different than the resonant frequency of the at least one sensor, to a receiver portion of the at least one sensor disposed on an opposed side of the fluid delivery line.

In step 182, the signal received by the at least one sensor is converted from analog to digital using the analog-to-digital converter. In step 184, a determination is made using the at least one processor whether air, fluid, or some combination thereof is in the fluid delivery line based on the converted digital signal. In step 186, if the determination is made in step 184 that air is disposed in the fluid delivery line, the at least one processor turns on an alarm to indicate to a user that air is disposed in the fluid delivery line. In one example, if the determination is made in step 184 that air is disposed in the fluid delivery line, then in step 186 the at least one processor turns on the alarm and shuts down the infusion system to stop the delivery of infusion fluid through the fluid delivery line. In other examples, the method 170 may be altered to vary the order or substance of any of the steps, to delete one or more steps, or to add one or more steps.

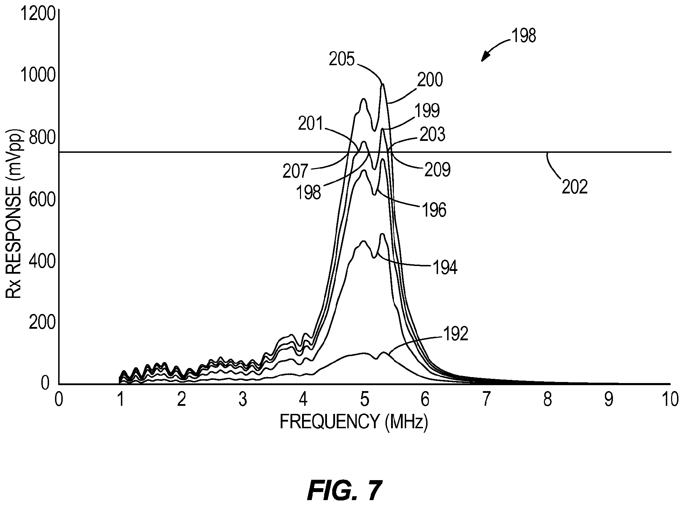

Figure 7 is a graph 190 illustrating five different curves 192, 194, 196, 198, and 200 showing for five different illustrative sensors, which could each be tried for the sensor 116 in the infusion system of Figure 1, how their respective signal strength varies as their modified frequency varies. The frequency performance for each sensor is plotted on the X-axis of the graph 190. The signal output of each sensor is plotted on the Y-axis of the graph 190. The electronic detection device 118 of Figure 1 requires a minimum useful signal strength of greater than or equal to 100 mVpp. The electronic detection device 118 of Figure 1 is over-saturated when the output of any of the sensors exceeds the saturation level 202 which is approximately 750 mVpp.

Curve 192 has a sensor output of below the minimum useful signal of 100 mVpp no matter how the modified frequency is varied. As a result this sensor should not be used because the signal is too weak. Curve 194 has a sensor output of below the minimum useful signal of 100 mVpp at some modified frequencies, and a sensor output of greater than or equal to the minimum useful signal of 100 mVpp at other modified frequencies yet the entire curve is below the saturation level 202 of 750 mVpp. As a result, this sensor can be used at any modified frequency which results in a signal output of greater than or equal to the minimum useful signal of 100 mVpp which is in the approximate range of between 4.3 MHz to 5.7 MHz as shown by curve 194. Curve 196 has a sensor output of below the minimum useful signal of 100 mVpp at some modified frequencies, and a sensor output of greater than or equal to the minimum useful signal of 100 mVpp at other modified frequencies yet the entire curve is below the saturation level 202 of 750 mVpp. As a result, this sensor can be used at any modified frequency which results in a signal output of greater than or equal to the minimum useful signal of 100 mVpp which is in the approximate range of between 4.2 MHz to 5.8 MHz as shown by curve 196.

Curve 198 has a sensor output of below the minimum useful signal of 100 mVpp at some modified frequencies, and a sensor output of greater than or equal to the minimum useful signal of 100 mVpp at other modified frequencies. Additionally, curve 198 has a sensor output of below the saturation level 202 of 750 mVpp at some modified frequencies, and a sensor output of above the saturation level 202 of 750 mVpp at other modified frequencies. As a result, this sensor can be used at any modified frequency which results in a signal output of greater than or equal to the minimum useful signal of 100 mVpp and results in a signal output of less than the saturation level 202 of 750 mVpp which is in the approximate range of between 3.4 MHz to 5.9 MHz as shown by curve 198. In order to obtain a modified frequency which results in a signal output of greater than or equal to the minimum useful signal of 100 mVpp and results in a signal output of less than the saturation level 202 of 750 mVpp, Applicants have discovered that the modified frequency for curve 198 needs to be within ± 36 % from the resonant frequency 199 of 5.3 MHz and within ± 31 % from the minimum or maximum saturation frequencies 201 and 203 which result in the saturation level of 750 mVpp. Preferably, the highest modified frequency is selected which results in a signal output as high as possible without exceeding the saturation level and is above the minimum useful signal strength.

Curve 200 has a sensor output of below the minimum useful signal of 100 mVpp at some modified frequencies, and a sensor output of greater than or equal to the minimum useful signal of 100 mVpp at other modified frequencies. Additionally, curve 200 has a sensor output of below the saturation level 202 of 750 mVpp at some modified frequencies, and a sensor output of above the saturation level 202 of 750 mVpp at other modified frequencies. As a result, this sensor can be used at any modified frequency which results in a signal output of greater than or equal to the minimum useful signal strength of 100 mVpp and results in a signal output of less than the saturation level 202 of 750 mVpp which is in the approximate range of between 3.5 MHz to 6.0 MHz as shown by curve 200. In order to obtain a modified frequency which results in a signal output of greater than or equal to the minimum useful signal of 100 mVpp and results in a signal output of less than the saturation level 202 of 750 mVpp, Applicants have discovered that the modified frequency for curve 200 needs to be within ± 34 % from the resonant frequency 205 of 5.3 MHz and within ± 27 % from the minimum or maximum saturation frequencies 207 and 209 which result in the saturation level of 750 mVpp. Preferably, the highest modified frequency is selected which results in a signal output as high as possible without exceeding the saturation level and which is above the minimum useful signal strength. In one embodiment, this modified frequency may be chosen so that the signal output is within 5% of the saturation level. In another example, this modified frequency may be chosen so that the signal output is within 10% of the saturation level. In other examples, varied modified frequencies may be chosen.

In other examples, other sensors may be used which have different sensor signal strength performance at varied modified frequencies. In one example, a sensor may be used which has a modified frequency of within ± 50 % from the resonant frequency and within ± 50 % from a saturation frequency. In another example, a sensor may be used which has a modified frequency of within ± 40 % from the resonant frequency and within ± 40 % from a saturation frequency. In another example, a sensor may be used which has a modified frequency of within ± 30 % from the resonant frequency and within ± 30 % from a saturation frequency. In another example, a sensor may be used which has a modified frequency of within ± 20 % from the resonant frequency and within ± 20 % from a saturation frequency. In another example, a sensor may be used which has a modified frequency of within ± 10 % from the resonant frequency and within ± 10 % from a saturation frequency.

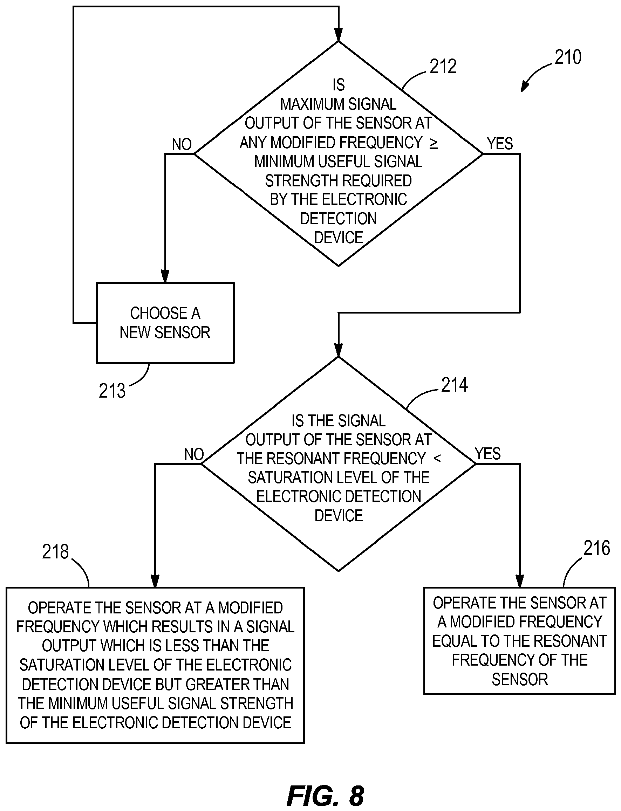

Figure 8 illustrates a flowchart of a method 210 for determining the modified frequency of an infusion system. The method 210 may utilize the infusion system of Figure 1. In other examples, the method 210 may utilize varying systems. The method 210 may be incorporated into any of the other methods disclosed herein including the methods illustrated in Figures 5 and 6 of this disclosure. In step 212, a determination is made as to whether the maximum signal output of the sensor at any modified frequency is greater than or equal to the minimum useful signal strength required by the electronic detection device. If the determination is made in step 212 that the maximum signal output of the sensor at any modified frequency is not greater than or equal to the minimum useful signal strength then in step 213 a new sensor is chosen and then step 212 is repeated until the determination is made in step 212 that the maximum signal output of the sensor at any modified frequency is greater than or equal to the minimum useful signal strength. Once the determination is made in step 212 that the maximum signal output of the sensor at any modified frequency is greater than or equal to the minimum useful signal strength, then the method proceeds from step 212 to step 214.

In step 214, a determination is made as to whether the signal output of the sensor at the resonant frequency is less than the saturation level of the electronic detection device. If the determination is made in step 214 that the signal output of the sensor at the resonant frequency is less than the saturation level of the electronic detection device, then the method proceeds to step 216. In step 216, the sensor is operated at a modified frequency which is equal to the resonant frequency of the sensor.

If the determination is made in step 214 that the signal output of the sensor at the resonant frequency is not less than the saturation level of the electronic detection device, then the method proceeds to step 218. In step 218, the sensor is operated at a modified frequency which results in a signal output which is less than the saturation level of the electronic detection device but greater than the minimum useful signal strength of the electronic detection device. Preferably, in step 218 the sensor is operated at the highest modified frequency which results in a signal output as high as possible without exceeding the saturation level of the electronic detection device and which is above the minimum useful signal strength of the electronic detection device. In one example of step 218, the sensor may be operated at a modified frequency within 5% of the saturation level. In another example of step 218, the sensor may be operated at a modified frequency within 10% of the saturation level. In other examples of step 218, the sensor may be operated at varied modified frequencies. In other examples, the method 210 may be altered to vary the order or substance of any of the steps, to delete one or more steps, or to add one or more steps.

One or more examples of the disclosure may improve the accuracy and reliability of the detection of air in infusion systems. One or more examples of the disclosure may be incorporated during calibration of one or more components of the infusion system. One or more examples of the disclosure may be done in the field without having to replace existing sensors of the infusion system by performing a field service procedure. This reduces the cost of sensor replacement and reduces the amount of replacement parts that must be kept in inventory. The disclosure also can accommodate greater variability and thus increases the yield of sensor assemblies that can be used.

The Abstract is provided to allow the reader to quickly ascertain the nature of the technical disclosure. It is submitted with the understanding that it will not be used to interpret or limit the scope or meaning of the claims. In addition, in the foregoing Detailed Description, it can be seen that various features are grouped together in various examples for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separately claimed subject matter.

While particular aspects of the present subject matter described herein have been shown and described, it will be apparent to those skilled in the art that, based upon the teachings herein, changes and modifications may be made without departing from the subject matter described herein and its broader aspects and, therefore, the invention encompasses within its scope all such changes and modifications as are within the scope of the appended claims.

## Claims

1. A method of arranging an infusion system, the infusion system comprising:
a fluid delivery line;
an analog-to-digital converter;
and at least one ultrasonic sensor configured to, in use, be electronically connected to the analog-to-digital converter, be disposed adjacent to the fluid delivery line and transmit and receive a signal to detect whether there is air in the fluid delivery line,
wherein the at least one ultrasonic sensor is provided to have a maximum signal output at any modified frequency which is greater than or equal to a minimum useful signal strength of the analog-to-digital converter; and
a pump for pumping infusion fluid through the fluid delivery line adjacent to the at least one ultrasonic sensor; and
wherein the analog-to-digital converter is configured to convert the signal received by the at least one sensor from analog to digital,
the method comprising:
determining a resonant frequency of the at least one ultrasonic sensor;
determining a saturation level of the analog-to-digital converter;
providing at least one processor configured to operate the at least one ultrasonic sensor;
determining whether operating the at least one sensor at the determined resonant frequency of the at least one sensor results
in an amplitude of an output of the signal transmitted from the at least one sensor which is lower than said determined saturation level of the analog-to-digital converter, or
in said amplitude being greater than or equal to said determined saturation level of the analog-to-digital converter; and
on the basis of said determination:
if the amplitude is determined as being lower than said determined saturation level of the analog-to-digital converter,
then selecting, as the frequency of operation of the at least one sensor, said determined resonant frequency of the at least one sensor; and
if the amplitude is determined as being greater than or equal to said determined saturation level of the analog-to-digital converter,
then selecting, as the frequency of operation of the at least one sensor, a modified frequency which is different than said determined resonant frequency of the at least one sensor in order to reduce the amplitude of the output of the signal transmitted from the at least one sensor to a level which is lower than said determined saturation level of the analog-to-digital converter to avoid over-saturating the analog-to-digital converter and greater than the minimum useful strength of the analog-to-digital converter,
wherein the at least one processor is configured to operate the at lest one sensor at the selected frequency of operation, and
wherein the method comprises programming the at least one processor to operate the at least one sensor at said modified frequency if the amplitude is determined as being greater than or equal to said determined saturation level of the analog-to-digital converter.

2. The method of claim 1, wherein the at least one processor is configured to determine whether air is in the fluid delivery line based on the converted digital signal.

3. The method of claim 1, wherein the step of determining the resonant frequency of the at least one sensor is carried out during calibration of the at least one sensor.

4. The method of claim 1, including a step of disposing the at least one ultrasonic sensor adjacent to the fluid delivery line of the infusion system, wherein said step comprises disposing a transmitter portion of the at least one sensor and a receiver portion of the at least one sensor on opposed sides of the fluid delivery line.

5. The method of claim 4, wherein the transmitter portion of the at least one sensor is configured to transmit the signal, while operating at the modified frequency, through the fluid delivery line, and the receiver portion of the at least one sensor is configured to receive the signal.

## Patentansprüche

1. Verfahren zum Anordnen eines Infusionssystems, wobei das Infusionssystem umfasst:
eine Fluidabgabeleitung;
einen Analog-Digital-Wandler;
und mindestens einen Ultraschallsensor, der konfiguriert ist, in Verwendung elektrisch mit dem Analog-Digital-Wandler verbunden zu sein, angrenzend an die Fluidabgabeleitung angeordnet zu sein und ein Signal zu senden und zu empfangen, um zu detektieren, ob Luft in der Fluidabgabeleitung ist,
wobei der mindestens eine Ultraschallsensor bereitgestellt ist, eine maximale Signalausgabe bei einer beliebigen modifizierten Frequenz aufzuweisen, die größer als oder gleich einer minimalen nützlichen Signalstärke des Analog-Digital-Wandlers ist; und
eine Pumpe zum Pumpen von Infusionsfluid durch die Fluidabgabeleitung, die an den mindestens einen Ultraschallsensor angrenzt; und
wobei der Analog-Digital-Wandler konfiguriert ist, das Signal, das von dem mindestens einen Sensor empfangen ist, von analog auf digital umzuwandeln,
wobei das Verfahren umfasst:
Ermitteln einer Resonanzfrequenz des mindestens einen Ultraschallsensors;
Ermitteln eines Sättigungslevels des Analog-Digital-Wandlers;
Bereitstellen mindestens eines Prozessors, der konfiguriert ist, den mindestens einen Ultraschallsensor zu betreiben;
Ermitteln, ob den mindestens einen Sensor bei der ermittelten Resonanzfrequenz des mindestens einen Sensors zu betreiben, darin resultiert, dass
eine Amplitude eines Ausgangs des Signals, das von dem mindestens einen Sensor gesendet wird, niedriger als das ermittelte Sättigungslevel des Analog-Digital-Wandlers ist, oder
die Amplitude größer als oder gleich dem ermittelten Sättigungslevel des Analog-Digital-Wandlers ist; und
auf der Basis der Ermittlung:
falls die Amplitude ermittelt ist, niedriger als das ermittelte Sättigungslevel des Analog-Digital-Wandlers zu sein,
dann, Auswählen, als die Betriebsfrequenz des mindestens einen Sensors, der ermittelten Resonanzfrequenz des mindestens einen Sensors; und,
falls die Amplitude ermittelt ist, größer als oder gleich dem ermittelten Sättigungslevel des Analog-Digital-Wandlers zu sein,
dann Auswählen, als die Betriebsfrequenz des mindestens einen Sensors, einer modifizierten Frequenz, die sich von der ermittelten Resonanzfrequenz des mindestens einen Sensors unterscheidet, um die Amplitude des Ausgangs des Signals, das von dem mindestens einen Sensor gesendet wird, auf ein Level zu verringern, das niedriger als das ermittelte Sättigungslevel des Analog-Digital-Wandlers ist, um Übersättigung des Analog-Digital-Wandlers zu vermeiden, und größer als die minimale nützliche Stärke des Analog-Digital-Wandlers ist, wobei der mindestens eine Prozessor konfiguriert ist, den mindestens einen Sensor bei der ausgewählten Betriebsfrequenz zu betreiben, und
wobei das Verfahren umfasst, den mindestens einen Prozessor zu programmieren, den mindestens einen Sensor bei der modifizierten Frequenz zu betreiben, falls die Amplitude ermittelt ist, größer als oder gleich dem ermittelten Sättigungslevel des Analog-Digital-Wandlers zu sein.

2. Verfahren nach Anspruch 1, wobei der mindestens eine Prozessor konfiguriert ist, basierend auf dem umgewandelten Digitalsignal zu ermitteln, ob Luft in der Fluidabgabeleitung ist.

3. Verfahren nach Anspruch 1, wobei der Schritt zum Ermitteln der Schwingungsfrequenz des mindestens einen Sensors während Kalibrierung des mindestens einen Sensors umgesetzt ist.

4. Verfahren nach Anspruch 1, beinhaltend einen Schritt zum Anordnen des mindestens einen Ultraschallsensors angrenzend an die Fluidabgabeleitung des Infusionssystems, wobei der Schritt umfasst, einen Senderabschnitt des mindestens einen Sensors und einen Empfängerabschnitt des mindestens einen Sensors an gegenüberliegenden Seiten der Fluidabgabeleitung anzuordnen.

5. Verfahren nach Anspruch 4, wobei der Senderabschnitt des mindestens einen Sensors konfiguriert ist, das Signal, während er bei der modifizierten Frequenz betrieben wird, durch die Fluidabgabeleitung zu senden, und der Empfängerabschnitt des mindestens einen Sensors konfiguriert ist, das Signal zu empfangen.

## Revendications

1. Procédé d'aménagement d'un système de perfusion, le système de perfusion comprenant :
un tube de fourniture de fluide ;
un convertisseur analogique-numérique ;
et au moins un capteur ultrasonique configuré pour, en service, être connecté au plan électronique au convertisseur analogique-numérique, être disposé adjacent au tube de fourniture de fluide et transmettre et recevoir un signal afin de détecter s'il y a ou non de l'air dans le tube de fourniture de fluide,
dans lequel le au moins un capteur ultrasonique est fourni pour avoir une sortie de signal maximale à une fréquence modifiée quelconque qui soit supérieure ou égale à une intensité de signal minimale utile du convertisseur analogique-numérique ; et
une pompe pour pomper du fluide de perfusion à travers le tube de fourniture de fluide adjacent à le au moins un capteur ultrasonique ; et
dans lequel le convertisseur analogique-numérique est configuré pour convertir le signal reçu par le au moins un capteur d'analogique à numérique,
le procédé comprenant :
la détermination d'une fréquence de résonance du au moins un capteur ultrasonique ;
la détermination d'un niveau de saturation du convertisseur analogique-numérique ;
la fourniture d'au moins un processeur configuré pour faire fonctionner le au moins un capteur ultrasonique ;
la détermination du fait que le fonctionnement ou non du au moins un capteur à la fréquence de résonance déterminée du au moins un capteur entraîne le fait
qu'une amplitude d'une sortie du signal transmis par le au moins un capteur est inférieure audit niveau de saturation déterminé du convertisseur analogique-numérique, ou
que ladite amplitude est supérieure ou égale audit niveau de saturation déterminé du convertisseur analogique-numérique ; et
sur la base de ladite détermination :
si l'amplitude est déterminée comme étant inférieure audit niveau de saturation déterminé du convertisseur analogique-numérique,
ensuite la sélection, comme la fréquence de fonctionnement du au moins un capteur, de ladite fréquence de résonance déterminée du au moins un capteur ; et
si l'amplitude est déterminée comme étant supérieure ou égale audit niveau de saturation déterminé du convertisseur analogique-numérique,
ensuite la sélection, comme la fréquence de fonctionnement du au moins un capteur, d'une fréquence modifiée qui est différente de ladite fréquence de résonance déterminée du au moins un capteur de manière à réduire l'amplitude de la sortie du signal transmis par le au moins un capteur à un niveau qui est inférieur audit niveau de saturation déterminé du convertisseur analogique-numérique afin d'éviter une sursaturation du convertisseur analogique-numérique et supérieur à l'intensité minimale utile du convertisseur analogique-numérique,
dans lequel le au moins un processeur est configuré pour faire fonctionner le au moins un capteur à la fréquence de fonctionnement sélectionnée et
dans lequel le procédé comprend la programmation du au moins un processeur afin de faire fonctionner le au moins un capteur à ladite fréquence modifiée si l'amplitude est déterminée comme étant supérieure ou égale audit niveau de saturation déterminé du convertisseur analogique-numérique.

2. Procédé selon la revendication 1, dans lequel le au moins un processeur est configuré pour déterminer s'il y a de l'air ou non dans le tube de fourniture d'air sur la base du signal numérique converti.

3. Procédé selon la revendication 1, dans lequel l'étape de détermination de la fréquence de résonance du au moins un capteur est effectuée au cours de l'étalonnage du au moins un capteur.

4. Procédé selon la revendication 1, incluant une étape de disposition du au moins un capteur électronique de manière adjacente au tube de fourniture de fluide du système de perfusion, dans lequel ladite étape comprend la disposition d'une partie de transmission du au moins un capteur et d'une partie de réception du au moins un capteur sur les côtés opposés du tube de fourniture de fluide.

5. Procédé selon la revendication 4, dans lequel la partie de transmission du au moins un capteur est configurée pour transmettre le signal, tout en fonctionnant à la fréquence modifiée, à travers le tube de fourniture de fluide, et la partie de réception du au moins un capteur est configurée pour recevoir le signal.
